# EUROPÄISCHE PATENTSCHRIFT

(11) **EP 2 844 076 B1**
(45) Veröffentlichungstag und Bekanntmachung des Hinweises auf die Patenterteilung: **27.04.2016**
(21) Anmeldenummer: 13726123.6
(22) Anmeldetag: 02.05.2013
(51) Int. Cl.: A23J 1/16

(54) **VERFAHREN ZUR GEWINNUNG VON PFLANZENPROTEINEN**
METHOD FOR OBTAINING PLANT PROTEINS
PROCÉDÉ D'EXTRACTION DE PROTÉINES VÉGÉTALES

(30) Priorität: 02.05.2012 EP 12166488
(43) Veröffentlichungstag der Anmeldung: 11.03.2015
(73) Patentinhaber: Lichner, Oskar, 63505 Langenselbold (DE); Lehmann, Thomas, 63505 Langenselbold (DE)
(72) Erfinder: Lichner, Oskar, 63505 Langenselbold (DE); Lehmann, Thomas, 63505 Langenselbold (DE)
(74) Vertreter: Jäger, Gerhard Fred
(86) Internationale Anmeldenummer: PCT/EP2013/059200
(87) Internationale Veröffentlichungsnummer: WO 2013/164430

(56) Entgegenhaltungen:
- WO-A1-2008/156795
- DE-C- 660 992
- DE-C- 960 239
- US-A- 5 275 732

## Beschreibung

Die Erfindung betrifft ein Verfahren zur Anreicherung, Gewinnung oder Entfernung von Pflanzenproteinen aus einer wäßrigen Flüssigkeit und die Verwendung von Wasserstoffgas oder einem Wasserstoffgas enthaltenden Gasgemisch in einem Verfahren zur Gewinnung von Pflanzenproteinen aus einer Flüssigkeit, zur Reinigung einer Flüssigkeit oder zur Abreicherung von Pflanzenproteinen aus einer Flüssigkeit.

Die Kartoffel enthält Proteine mit einer sehr ausgewogenen Aminosäurezusammensetzung, die der Zusammensetzung im Hühnereiweiß sehr nahe kommt. Das bei der Stärkegewinnung in großen Mengen anfallende Kartoffelfruchtwasser enthält etwa 0,5 bis 2,5 Gew.-% Proteine. Es besteht ein großes Interesse, Proteine aus solchen Flüssigkeiten zu gewinnen und nutzbar zu machen.

Die Abtrennung und Isolation von Proteinen aus Kartoffelfruchtwasser ohne sie auch nur teilweise negativ zu verändern oder zu denaturieren ist schwierig. Frisches Kartoffelfruchtwasser ist ein komplexes Gemisch aus Proteinen, Reste von Stärke, Mineralien, toxischen Glykoalkaloiden und monomeren und polymeren, sehr reaktiven Phenolverbindungen. Die Luftoxidation dieser Phenolverbindungen verursacht eine schnelle Verfärbung des Kartoffelfruchtwassers, es wird braun bis schwarz. Während dieses Oxidationsprozesses können die Proteine weiter reagieren, vernetzen und unerwünschte Nebenprodukte bilden.

Verbreitet sind Verfahren, die eine Abtrennung der Proteine über eine Ausfällung nach Koagulation erreichen. Koagulation erfolgt durch Wärme und/oder Verschiebung des pH-Wertes. Ein solches Verfahren ist beispielsweise in der DE102006050620 A1 beschrieben. Bei diesen Verfahren können nur denaturierte Proteine gewonnen werden, die ernährungsphysiologisch wenig geeignet und daher für lebensmitteltechnische und pharmazeutische Anwendungen ungeeignet sind. Außerdem müssen eventuell eingesetzte Hilfsreagenzien, wie organische Säuren oder Salze wieder abgetrennt werden. Die auf diese Weise gewonnenen Proteine verlieren durch die dabei einhergehende Denaturierung ihre wertvollen funktionalen Eigenschaften wie Löslichkeit, Emulgierfähigkeit, Schaumbildung, Fähigkeit Wasser zu binden und thermisch zu gelieren. Schonendere Arten das Produkt zu gewinnen, sind Membranverfahren (Ultrafiltration), die sich durch einen hohen Wirkungsgrad auszeichnen und technisch schon für andere Zwecke eingesetzt werden. Diese Verfahren sind aber sehr aufwändig.

Ein anderes Verfahren zur Gewinnung von Protein aus dem Fruchtwasser der Kartoffeln wird in der DE660992 C1 beschrieben. Mit Hilfe eines Gases wie Kohlendioxid wird Schaum erzeugt, in dem das Protein angereichert ist und mit dem Schaum abgetrennt werden kann. Ein solches Verfahren wird zur Anreicherung und Isolierung oberflächenaktiver Stoffe, darunter auch Proteine, in der DE960239 C1 beschrieben. Verfahren mit einer Anreicherung gelöster oberflächenaktiver Stoffe in einem Schaum sind unter den Begriffen Zerschäumung, Zerschäumungsanalyse oder im Englischen "adsorptive bubble separation" bekannt.

Bei allen genannten Verfahren enthält das gewonnene Kartoffelprotein bisher in der Regel einen nicht tolerierbaren Gehalt an Glykoalkaloiden, der einen Einsatz im Lebensmittelbereich ausschließt.

Wünschenswert ist es daher, ein Verfahren zu entwickeln, das die Gewinnung nativer Proteine ohne toxische Begleitstoffe ermöglicht.

Der hohe Gehalt an organischen Bestandteilen in Abwässern aus der Verarbeitung von Pflanzen und Pflanzenteilen, insbesondere aus der Kartoffelstärkeproduktion, stellt ein Problem dar. Die früher praktizierte Entsorgung via Tierverfütterung oder Verregnung auf landwirtschaftlichen Flächen ist nicht mehr zulässig. Wege zu einer Verringerung der organischen Abwasserbelastung und der Abwassermengen bei der Kartoffelverarbeitung und anderen Prozessen werden daher gesucht.

Aufgabe der Erfindung ist somit die Schaffung eines alternativen Verfahrens zur Gewinnung von reinen, nicht denaturierten, nativen Proteinen aus Flüssigkeiten, die pflanzliche Proteine enthalten, insbesondere aus Prozesswässern und Abwässern aus der Verarbeitung pflanzlicher Produkte. Eine weitere Aufgabe besteht darin, ein alternatives Verfahren bereit zu stellen, das den Anteil organischer Bestandteile in einem Abwasser oder Prozesswasser pflanzenverarbeitender Prozesse verringert.

Gelöst wurde die Aufgabe durch ein Verfahren mit den in Anspruch 1 beschriebenen Merkmalen.

Das Verfahren dient zur Anreicherung, Gewinnung oder Entfernung von Pflanzenproteinen aus einer wäßrigen Flüssigkeit, die gelöste oder emulgierte Pflanzenproteine enthält. In dem Verfahren werden durch elektrochemische Gasentwicklung oder mit Hilfe von Wasserstoffgas oder einem Wasserstoffgas enthaltenden Gasgemisch Gasblasen in der Flüssigkeit erzeugt. In dem bevorzugten Verfahren wird ein Schaum gebildet, der gelöste Pflanzenproteine enthält. In dem Schaum werden Pflanzenproteine angereichert. Der Schaum wird genutzt, um lösliche, nicht denaturierte Pflanzenproteine zu gewinnen. In der Regel wird zur Gewinnung von Pflanzenproteinen oder einem Produkt mit Pflanzenproteinen oder zur Reinigung der Flüssigkeit Schaum von der Flüssigkeit durch eine geeignete Vorrichtung getrennt und abgeführt, z.B. durch Schaumableiten oder Schaumentnahme. Vorteilhaft wird eine Vorrichtung mit einem Rohr, einem rohrartigen Gebilde oder einem Hohlkörper mit einer Eingangsöffnung und einer Ausgangsöffnung verwendet, worin der gebildete Schaum aufsteigen kann. Die Vorrichtung ist beispielsweise eine Elektrolyse-Zelle oder ein Elektrodenraum einer Elektrolyse-Zelle, die mit einem Schacht, einem Kanal, einem Rohr oder rohrartigen Gebilde verbunden ist oder einen Bereich aufweist, in dem der gebildete Schaum aufsteigen kann. Das Rohr oder die analogen Gebilde werden vorteilhaft so dimensioniert, dass ihr Durchmesser das Dreifache bis zu einem Zehntel des Elektrolyse-Zellendurchmessers beträgt. Schaumbildung und Schaumabführung (Trennung von Schaum von der Flüssigkeit in der Vorrichtung) führen zu einer Abreicherung der Flüssigkeit von Pflanzenproteinen, die sich im Gefäß oder Behälter der Schaumerzeugung befindet. Eine Schaumabführung (Schaumabtrennung) kann über einen Überlauf erfolgen. Die Protein-Abreicherung der Flüssigkeit wird auch für eine Reinigung der Flüssigkeit eingesetzt. Eine Schaumbehandlung einer Flüssigkeit umfaßt Schaumbildung und Schaumabführung.

Im Rahmen der vorliegenden Erfindung wird unter dem Begriff" Pflanzenprotein" jedes Protein verstanden, das pflanzlichen Ursprungs ist, d.h. in Pflanzen, Pflanzenteilen oder anderen Pflanzenprodukten enthalten ist. Pflanzenprodukte sind z.B. Obst, Früchte, Beeren, Nüsse, Pflanzenkerne, Samen, Knollen, Wurzeln. Flüssigkeiten mit gelösten Pflanzenproteinen entstehen beispielsweise bei der Verarbeitung von stärkehaltigen Pflanzenprodukten, z.B. Getreide und Kartoffeln. Flüssigkeiten mit gelösten Pflanzenproteinen fallen insbesondere bei der Verarbeitung von Agrar- oder Nutzpflanzen oder deren Pflanzenprodukte an. Agrar- oder Nutzpflanzen oder deren Pflanzenprodukte sind beispielsweise Mais, Reis, Gerste, Weizen, Roggen, Hafer, Hirse, Soja, Tapioka, Topinambur, Kartoffeln und Süßkartoffeln, Spargel, Schwarzwurzel, Rote Beete, Cassava, Maniok, Tannia, Canna, Yamswurzel, Pfeilwurzel, Wasserbrotwurzel, Emmer, Raps, Gurken, Melonen, Kürbis, Nüsse, Erdnüsse, Sonnenblumenkerne, Zuckerrohr, Zuckerrübe, Karotte, Möhre, Kohl, Kohlrabi, Äpfel oder Weintrauben.

Unter dem Begriff "Protein" wird im Rahmen der vorliegenden Erfindung jedes Molekül verstanden, das mindestens zehn Aminosäuren umfasst. Damit sind im Rahmen der vorliegenden Erfindung auch Peptide, Oligopeptide und Polypeptide vom Begriff "Protein" umfasst.

Flüssigkeiten mit gelösten Pflanzenproteinen sind insbesondere Pflanzenfruchtwasser, z.B. Kartoffelfruchtwasser. Unter dem Begriff" Pflanzenfruchtwasser" wird dabei jede Flüssigkeit verstanden, die gelöstes Pflanzenmaterial oder gelöste pflanzliche Stoffe enthält. Der Begriff" Pflanzenfruchtwasser" umfaßt Prozesswasser aus der Verarbeitung von Pflanzen, Pflanzenteilen oder Pflanzenprodukten. Pflanzenfruchtwasser kann aus Pflanzenabfällen von Agrar- oder Nutzpflanzen oder deren Pflanzenprodukte, z.B. Pflanzenschalen oder Pflanzenreste, insbesondere Kartoffelschalen, gewonnen werden. Pflanzenfruchtwasser kann durch Pressen, Extraktion oder durch Waschen von Pflanzenmaterial, insbesondere von geschnittenen oder zerkleinerten Pflanzen, Pflanzenteilen oder Pflanzenprodukten, erhalten werden. Als Lösemittel, Extraktionsmittel oder Waschflüssigkeit wird bevorzugt Wasser eingesetzt. Dem Wasser können Hilfsmittel oder andere Lösemittel zugesetzt sein. Es kann im Rahmen der vorliegenden Erfindung jedoch jedes Lösungsmittel eingesetzt werden, das dem Fachmann als für den erfindungsgemäßen Zweck geeignet bekannt ist. Desweiteren kann Pflanzenfruchtwasser durch jedes Verfahren hergestellt werden, das dem Fachmann als geeignet bekannt ist.

Die eingesetzten Flüssigkeiten enthalten z.B. 0,01 bis 10 Gew.-% Proteine, insbesondere 0,1 bis 5 Gew.-% Proteine oder 0,1 bis 3 Gew.-% Proteine. Flüssigkeiten mit niedrigem Proteingehalt sind in dem Verfahren verwendbar. Typisch ist der Einsatz von Flüssigkeiten mit einem Proteingehalt im Bereich von 0,5 bis 3 Gew.-% Proteine bzw. Protein.

In einzelnen Fällen kann eine Verdünnung der Flüssigkeit vorteilhaft sein. Beispielsweise wird die Flüssigkeit 1:5 oder 1:10 mit Wasser verdünnt. Eine Verdünnung der eingesetzten Flüssigkeit kann zu einer Verbesserung der Reinheit des Produktes der Schaumbehandlung führen.

Die Flüssigkeit, insbesondere Pflanzenfruchtwasser, z.B. Kartoffelfruchtwasser, kann ein Stabilisierungsmittel enthalten. Dem Kartoffelfruchtwasser kann beispielsweise ein Stabilisierungsmittel zugefügt werden um chemische Veränderungen wie Zersetzungsreaktionen bei der Lagerung zu vermeiden. Die Verwendung von Wasserstoffgas oder Wasserstoffgas enthaltenden Gasgemischen ist bei Verfahren mit Anreicherung gelöster Pflanzenproteine aus wäßrigen Medien, insbesondere von proteinhaltigen Lösungen, besonders vorteilhaft.

Als Zerschäumung wird ein Verfahren bezeichnet, in dem in mindestens einem Schritt eine Schaumbildung in einer wäßrigen Flüssigkeit, die gelöste oberflächenaktive Stoffe wie Proteine enthält, eingesetzt wird, um einen mit diesen Stoffen angereicherten Schaum herzustellen.

Die Behandlung, Abtrennung und Aufarbeitung des Schaumes kann in dem Verfahren gemäß der Erfindung wie bei den herkömmlichen Verfahren der Zerschäumung erfolgen. Der Schaum für eine Zerschäumung wird beispielsweise durch Einleiten eines Gases in die Flüssigkeit gebildet. Feine Gasblasen können durch Sättigung der Flüssigkeit mit dem Gas unter Druck und anschließender Entspannung erzeugt werden, also durch Druckentspannung. In der Regel wird der gebildete Schaum abgetrennt, abgeführt oder abgeleitet. Die Zerschäumung kann im Batch-Verfahren oder kontinuierlich betrieben werden. Die Zerschäumung kann zur Abreicherung einer Flüssigkeit von enthaltenen gelösten Pflanzenproteinen, zu einer Anreicherung von Pflanzenproteinen oder zur Gewinnung von Pflanzenproteinen dienen. Vorteilhaft läßt man den Schaum in einem Rohr aufsteigen. Es bildet sich eine Schaumsäule, die Einfluß auf die Verteilung der Pflanzenproteine hat und eine Trennwirkung zwischen verschiedenen Pflanzenproteinen haben kann. Da die Gasblasen in der Schaumsäule adsorptiv wirken, spricht man auch von einer adsorptiven Zerschäumung. Verfahren der Zerschäumung sind beispielsweise in DE327976 C1, DE660992 C1 und DE960239 C1 beschrieben, worauf hiermit Bezug genommen wird. Die Behandlung, Abtrennung und Aufarbeitung des Schaumes kann in dem Verfahren gemäß der Erfindung wie bei den herkömmlichen Verfahren der Zerschäumung erfolgen.

Die Verwendung von Wasserstoffgas oder Wasserstoffgas enthaltenden Gasgemischen erleichtert die Produktabtrennung und ermöglicht eine schonendere Produktgewinnung. Es lassen sich bei Wasserstoffgas sehr kleine Gasblasen, insbesondere bei einer kathodischen Wasserstoffentwicklung, erzeugen, die zu einer effektiveren Anreicherung von Pflanzenproteinen im gebildeten Schaum führt. Bisher erzielte Ergebnisse legen nahe, dass Gasblasen aus Wasserstoff günstige Adsorptionseigenschaften an der Phasengrenzfläche zwischen Flüssigkeit und Gas aufweisen. Aber auch eine schonendere Abtrennung von Pflanzenproteinen zeichnen Wasserstoffgas und Wasserstoffgas enthaltende Gasgemische als schaumbildendes Gas bei schaumbildenden Verfahren zur Anreicherung oder Gewinnung von Pflanzenproteinen wie der Zerschäumung aus. Die reduktiven Bedingungen bei der Schaumbildung mit Wasserstoffgas schützen vor einer Schädigung des zu gewinnenden Produktes und von Stoffen in der Flüssigkeit durch Luftsauerstoff. Das ist besonders vorteilhaft bei Kartoffelfruchtwasser, das oxidationsempfindliche Substanzen mit Phenolgruppen (z.B. Polyphenole) enthält, deren Oxidationsprodukte eine Aufarbeitung der Flüssigkeit erschweren und zu einer Schwärzung der Flüssigkeit führen. Das durch Zerschäumen mit Wasserstoffgas gewonnene Produkt enthält daher weniger Verunreinigungen durch oxidative Abbau- oder Zersetzungsprodukte. Es können empfindliche Proteine ohne Schädigung gewonnen werden. Die Verwendung von Wasserstoffgas oder Wasserstoffgas enthaltenden Gasgemischen bei der Schaumbildung zur Anreicherung und Abtrennung von Pflanzenproteinen ist daher besonders vorteilhaft für die Gewinnung nativer Proteine. Die gewonnenen Proteine behalten in der Regel ihre Fähigkeit sich in Wasser zu lösen.

Zur Schaumbildung kann der Einsatz von Wasserstoffgas mit einem oder mehreren verschiedenen Gasen, gleichzeitig oder nacheinander, kombiniert werden. Beispielsweise können die Gase Stickstoff, Sauerstoff. Kohlendioxid neben Wasserstoff eingesetzt werden. Es können inerte Gase oder reaktive Gase zusätzlich eingesetzt werden. Beispielsweise kann die Schaumbildung mit Wasserstoffgas beginnen und mit einem anderen Gas fortgesetzt werden.

Flüssige Produkte mit Pflanzenprotein, die in dem Verfahren mit Schaumbildung gewonnen werden, können durch zusätzliche (ein- oder mehrmalige) Schaumbehandlung (Zerschäumung) weiter angereichert werden.

Vorteilhaft sind Verfahren mit einer Schaumerzeugung mittels elektrochemisch erzeugter Gase oder Gasgemische in der Flüssigkeit, insbesondere elektrochemisch erzeugtes Wasserstoffgas oder elektrochemisch erzeugte Wasserstoffgas enthaltende Gasgemische. In der Flüssigkeit elektrochemisch (elektrolytisch) erzeugte Gasblasen unterscheiden sich von herkömmlich erzeugten Gasblasen bei der Zerschäumung. Elektrolytisch erzeugte Gasblasen sind in der Regel sehr viel kleiner als bei einer Gaseinleitung und führen zu einer günstigen Schaumkonsistenz für eine Anreicherung und Abtrennung gelöster Pflanzenproteine. Prinzipiell können eine kathodische Wasserstoffentwicklung und eine anodische Sauerstoffentwicklung zur Gasblasenerzeugung genutzt werden. Wasserstoffgas und Sauerstoffgas unterscheiden sich jedoch im Adsorptionsverhalten. Überraschend wurde gefunden, dass bei elektrolytischer Wasserstoffgasblasenerzeugung bei der Proteingewinnung aus Kartoffelfruchtwasser Schäume mit einem geringen Gehalt an Glykoalkaloiden erhalten werden. Die Verwendung von Wasserstoffgas kann daher bei Flüssigkeiten mit organischen toxischen Verunreinigungen für eine Gewinnung von Pflanzenprotein-Produkten mit reduziertem Gehalt an toxischen Begleitstoffen genutzt werden. Zudem schützt Wasserstoffgas vor oxidativen Reaktionen, wie schon erwähnt wurde. Schaum mit Wasserstoffgas führt zu einer besseren Produktqualität bei der Gewinnung von Pflanzenproteinen. Bevorzugt wird eine kathodische Wasserstoffentwicklung zur Schaumbildung, insbesondere in geteilter Zelle.

Ein weiterer Gegenstand der Erfindung ist somit ein Verfahren zur Gewinnung oder Entfernung von Pflanzenproteinen aus einer wäßrigen Flüssigkeit, dadurch gekennzeichnet, dass elektrochemisch Gasblasen aus Wasserstoffgas oder einem Wasserstoffgas enthaltenden Gasgemisch in der Flüssigkeit erzeugt werden und ein Schaum gebildet wird, der gelöste Pflanzenproteine enthält, wobei der Schaum oder ein Teil des Schaumes von der Flüssigkeit getrennt wird und ein Produkt oder Zwischenprodukt mit Pflanzenproteinen gewonnen wird, das einen geringen Gehalt an toxischen Begleitstoffen aufweist. Das Verfahren ist besonders vorteilhaft mit Kartoffelfruchtwasser als Flüssigkeit oder mit einer Flüssigkeit, die Kartoffelproteine enthält. Hierbei wird ein Produkt oder Zwischenprodukt mit Pflanzenproteinen gewonnen, das einen geringen Gehalt an Glykoalkaloiden oder weniger als 100 ppm Glykoalkaloide, vorzugsweise weniger als 50 ppm Glykoalkaloide, besonders bevorzugt weniger als 10 ppm Glykoalkaloide, aufweist (1 ppm ist ein Gramm pro 10⁶ Gramm).

Das Verfahren zur Gewinnung oder Entfernung von Pflanzenproteinen aus einer wäßrigen Flüssigkeit wird ohne eine Ausfällung von Pflanzenproteinen durchgeführt. Bei der Verfahrensvariante mit elektrochemischer Gasblasenerzeugung in der Flüssigkeit handelt es sich somit um keine Elektroflotation, da die Elektroflotation immer zur Abtrennung von Partikeln dient.

Die elektrochemische Zelle (Elektrolyse-Zelle) kann eine ungeteilte oder geteilte Zelle sein. Vorzugsweise weist die elektrochemische Zelle mindestens ein Rohr oder rohrartiges Gebilde auf, in dem gebildeter Schaum aufsteigen kann. Das Rohr oder rohrartige Gebilde ist in der Regel vertikal an der elektrochemischen Zelle angeordnet oder mit der elektrochemischen Zelle verbunden. Allgemein ist eine elektrochemische Zelle vorteilhaft, die ein hohles Teil oder hohles Gebilde enthält oder mit einem hohlen Teil mit unterer und oberer Öffnung verbunden ist, worin gebildeter Schaum aufsteigen kann.

Das Rohr oder rohrartige Gebilde, in dem gebildeter Schaum aufsteigen kann, hat beispielsweise eine Länge von mindestens 0,5 m, vorteilhaft mindestens 1 m, besonders vorteilhaft mindestens 2 m. Das obere Ende des Rohres oder rohrartigen Gebildes ist beispielsweise als Überlauf gestaltet. Die Länge des Rohres oder rohrartigen Gebildes hat Einfluß auf die Konsistenz und Zusammensetzung des überlaufenden Schaumes. Vom Überlauf können Probenfraktionen (Schaumproben) für eine fraktionierte Pflanzenproteingewinnung gesammelt werden. Vom Überlauf abfließender Schaum wird zur Pflanzenproteingewinnung weiter verarbeitet. In der Regel wird der Schaum zunächst gebrochen. Z.B. wird überlaufender Schaum in einen Schaumbrecher (Vorrichtung zur Schaumzerstörung) oder in einen Sammelbehälter geleitet. Der Schaum wird in eine Flüssigkeit überführt, die gegenüber der ursprünglich eingesetzten Flüssigkeit einen höheren Proteingehalt hat. Dieses flüssige Produkt (Proteinlösung) kann zur Gewinnung enthaltener Pflanzenproteine oder zu einem Pflanzenproteinkonzentrat weiter aufgearbeitet werden. Geeignete Verfahren zur Aufarbeitung solcher Proteinlösungen sind dem Fachmann bekannt.

Bei der elektrolytischen Wasserstoff-Erzeugung in der zu behandelnden Flüssigkeit werden in der Regel sehr kleine Gasblasen aus Wasserstoff gebildet. Die erzeugten Gasblasen haben im Allgemeinen einen Durchmesser von unter 50 µm. Es lassen sich Gasblasen mit einem Durchmesser unter 30 µm, insbesondere unter 25 µm, auf einfache Weise herstellen. Die elektrochemisch bevorzugt in der zu behandelnden Flüssigkeit erzeugten Gasblasen haben vorzugsweise einen Durchmesser im Bereich von 1 bis 50 µm, besonders bevorzugt im Bereich von 1 bis 30 µm, insbesondere im Bereich von 5 bis 25 µm. Kleine Gasblasen sind sehr vorteilhaft. Sie verbessern u.a. die Proteinanreicherung im Schaum.

Für die elektrochemische Erzeugung von Wasserstoffgas sind im Allgemeinen als Kathode Elektroden mit Elektrodenmaterialien verwendbar, die für eine kathodische Wasserstoffentwicklung geeignet sind. Sie enthalten z.B. Platin, Nickel, Eisen, Iridium, Ruthenium, Palladium oder leitfähige kohlenstoffhaltige Materialien. Kathodenmaterialien sind beispielsweise Platin, Glaskohlenstoff (glassy carbon), Grafit, leitfähiger Diamant, insbesondere Bor-dotierter Diamant (BDD) oder leitfähige kohlenstoffhaltige Materialien, insbesondere Carbon Nanotubes und Ruß. Platin-Kathoden sind beispielsweise platinbeschichtete Titan-Elektroden. Grafit wird z.B. in Form von flächigen flachen Gebilden (z.B. Platten), einer planaren mikrostrukturierten Grafitschicht, Formkörpern oder Teilchen als Elektrode eingesetzt. Eine Grafit-Teilchen-Elektrode ist z.B. eine Grafitpartikelschüttung. Die Partikeldurchmesser einer Grafitpartikelschüttung liegen beispielsweise im Bereich von 0,1 bis 5 mm. Die Partikel-Elektrode kann als Festbett- oder Wirbelbett-Elektrode ausgebildet sein. Kohlenstoffhaltige Kathodenmaterialien wie Grafit, leitfähiger Diamant und Kohlenstoff-Elektroden haben sich als vorteilhaft erwiesen.

Der Elektrolyt, insbesondere der Katholyt (Elektrolyt im Kathodenraum), besitzt bevorzugt einen pH-Wert im Bereich von 4 bis 9 bevorzugter von 4 bis 8,8, weiter bevorzugt von 4 bis 8,5, noch bevorzugter von 4,5 bis 8,2, besonders bevorzugt von 4,5 bis 8,0 und am meisten bevorzugt von 4,8 bis 6,5. Die Flüssigkeiten, die Pflanzenproteine enthalten, können in der Regel direkt als Elektrolyt eingesetzt werden. Die eingesetzte Flüssigkeit, z. B. Kartoffelfruchtwasser, hat gewöhnlich einen pH-Wert im Bereich von pH 4 bis pH 7. Der pH-Wert der Flüssigkeit kann auf den gewünschten Wert eingestellt werden.

Der pH-Wert des Elektrolyten, insbesondere des Katholyten, kann während der Elektrolyse konstant gehalten oder verändert werden. In vielen Fällen bleibt der pH-Wert bei Batch-Betrieb und ungeteilter Zelle konstant. Ein pH-Gradient bei der Zerschäumung kann auf elegante Weise durch Nutzung der pH-Drift während der Elektrolyse in geteilter Zelle im Batchbetrieb realisiert werden, da der Katholyt im Laufe der Elektrolyse alkalischer wird.

Gasmenge, Gasblasenmenge und Gasflussrate können durch Veränderung der Stromdichte an der Kathode eingestellt werden.

Vorteilhaft werden in dem Verfahren eine oder mehrere geteilte Elektrolyse-Zellen eingesetzt, die diskontinuierlich (Batch-Betrieb) oder kontinuierlich (Durchfluss-Betrieb) betrieben werden. Die geteilte Elektrolysezelle kann insbesondere eine Durchflusszelle sein.

Die Elektrolyse erfolgt in der Regel bei einer Temperatur im Bereich von 0° C bis 40° C, vorzugsweise im Bereich von 10° C bis 30° C, insbesondere im Bereich von 15° C bis 30° C oder im Bereich von 15° C bis 25° C. Beispielsweise erfolgt die Elektrolyse bei Raumtemperatur.

Vorteilhaft ist die Elektrolysezelle so dimensioniert, dass die Höhe der Elektrolysezelle die Breite oder den Durchmesser der Elektrolysezelle um ein Vielfaches übertrifft. Es werden vorteilhaft Elektroden verwendet, deren Höhe-zu-Breite-Verhältnis (bei flachen Elektroden) oder Höhe-zu-Durchmesser-Verhältnis (bei zylindrischen Elektroden) größer als 1 ist. Besonders vorteilhaft ist ein Höhe-zu-Breite-Verhältnis oder Höhe-zu-Durchmesser-Verhältnis der Elektroden von mindestens 10. Eine solche Dimensionierung der Elektroden schafft eine größere Gasblasenstrecke entlang der Kathode.

Bei einer kontinuierlich betriebenen, geteilten Elektrolysezelle werden in der Regel Kathodenraum und Anodenraum mit Elektrolyt aus Behältern mittels Pumpen versorgt. Vorteilhaft ist ein Betrieb im Kreislauf zwischen Behälter und Elektrodenraum. In der Regel dienen Flüssigkeiten wie Pflanzenfruchtwasser, insbesondere Kartoffelfruchtwasser, als Katholyt. Solche Flüssigkeiten, insbesondere nach Schaumbehandlung und Proteinabreicherung im Kathodenraum, dienen vorzugsweise als Anolyt. Es kann vorteilhaft sein, andere Flüssigkeiten als Anolyt zu verwenden, je nach dem welche Anodenreaktion gewählt wird.

Die Kathodenreaktion erfolgt z.B. bei einer Stromdichte von 5-15 mA/cm². Die Elektrolysezeit beträgt z.B. 2 bis 3 Stunden bei Batchbetrieb.

Eine Alternative zu einer anodischen Sauerstoffentwicklung oder anodischen Oxidation organischer Stoffe ist eine anodische Wasserstoffoxidation als Anodenreaktion. Eine Kombination einer kathodischen Wasserstofferzeugung zur Schaumerzeugung mit einer anodischen Wasserstoffoxidation bietet Vorteile hinsichtlich des Energiebedarfes. Es wird vorzugsweise eine geteilte Zelle verwendet. Besonders vorteilhaft zur anodischen Wasserstoffoxidation sind Gasdiffusionselektroden. Eine geeignete Gasdiffusionselektrode wird z.B. durch Verpressen von platiniertem Ruß und Polytetrafluorethylen mit einer Kationenaustauschermembran hergestellt, wobei beispielsweise eine 40-50 µm dicke Beschichtung auf der Kationenaustauschermembran erzeugt wird. Solche Gasdiffusionselektroden und die Durchführung einer anodischen Wasserstoffoxidation sind in der EP0800853 A2 beschrieben, worauf hiermit Bezug genommen wird. Durch Kopplung der kathodischen Wasserstofferzeugung für eine Schaumbildung mit einer anodischen Wasserstoffoxidation kann der Energieverbrauch deutlich gesenkt werden. Ein weiterer Gegenstand der Erfindung ist somit ein Verfahren, in dem für eine Schaumbildung in einer Flüssigkeit mit Pflanzenproteinen, z.B. ein Prozesswasser oder Abwasser, in einer elektrochemischen Zelle, vorzugsweise einer geteilten Zelle, kathodisch Wasserstoffgas in der Flüssigkeit erzeugt und als Gegenreaktion an der Anode Wasserstoff oxidiert wird.

Vorteilhaft wird Wasserstoffgas, das elektrolytisch erzeugt wird, wobei in der Regel die kathodische Wasserstoffgasblasenbildung in der zu behandelnden Flüssigkeit unter einer Schaumbildung erfolgt, für eine Behandlung einer Flüssigkeit innerhalb oder außerhalb der Elektrolyse-Zelle genutzt. Beispielsweise kann das zunächst elektrochemisch erzeugte Wasserstoffgas der Elektrolysezelle entnommen werden und in Form feiner Gasblasen wieder in die Elektrolysezelle bzw. in den Kathodenraum oder einen externen Behälter mit zu behandelnder Flüssigkeit (für eine Zerschäumung außerhalb der Elektrolysezelle) eingespeist werden. Die Einspeisung oder Rückführung des Wasserstoffgases kann bei laufender Elektrolyse oder abgeschalteter Elektrolyse erfolgen. Bei der Einspeisung oder Rückführung des Wasserstoffgases werden z.B. Gasblasenerzeuger eingesetzt. Als Gasblasenerzeuger kann allgemein jede dem Fachmann dazu geeignet erscheinende Vorrichtung eingesetzt werden. Geeignete Glasblasenerzeuger sind beispielsweise Fritten aus Metall oder Glas aus denen die Gasblasen herausperlen, Injektoren, die gegen eine Prallplatte gerichtet sein können, oder nach dem Venturi-Prinzip arbeitende Injektoren. Die Injektoren könne auch mit einem statischen Mischer kombiniert werden, der den Gasstrom aus dem Injektor in kleine Gasblasen zerteilt und diese möglichst homogen verteilt. Zur Gasblasenerzeugung kann vorteilhaft eine Druckentspannung dienen. Bei Einsatz der Druckentspannung kann eine Rückführung des Wasserstoffgases entfallen.

Einer Rückführung oder Wiederverwendung von Wasserstoffgas, insbesondere von elektrolytisch erzeugtem Wasserstoffgas, zur Schaumbehandlung (Zerschäumung) einer Flüssigkeit mit gelösten Pflanzenproteinen innerhalb oder außerhalb einer elektrochemischen Zelle wird die Verwendung von anfallendem Wasserstoffgas für eine anodische Wasserstoffoxidation vorgezogen, da die elektrolytische Gasblasenerzeugung anderen Verfahren zur Gasblasenerzeugung bei Zerschäumung überlegen ist.

In dem Verfahren mit Schaumbildung zur Anreicherung, Gewinnung oder Abtrennung von in einer Flüssigkeit gelösten oder emulgierten Pflanzenproteinen kann Wasserstoffgas kontinuierlich (z.B. galvanostatisch oder potentiostatisch) oder diskontinuierlich elektrolytisch erzeugt werden. Beispielsweise wird Wasserstoffgas in Pulsen erzeugt, vorzugsweise in der Flüssigkeit. In einem solchen Pulsbetrieb wird der Elektrolysestrom für eine bestimmte Zeit wiederholt ein- und ausgeschaltet. Solche Strompulse können von konstanter Dauer oder variabel in der Dauer sein. Vorteilhaft wird die Abfolge von Strompulsen mit Hilfe eines Steuergerätes oder einer Steuereinheit erzeugt. Das Steuergerät kann z.B. ein Pulsgenerator oder ein programmierbares Gerät sein. Die Länge eines Strompulses kann über eine Regeleinrichtung oder Sensorsteuerung bestimmt werden. Beispielsweise kann der Elektrolysestrom unterbrochen werden, wenn die Schaumbildung eine bestimmte Größe oder die Flüssigkeit eine bestimmte Eigenschaft erreicht haben. Solche Regelgrößen sind z.B. die Höhe einer Schaumsäule in einem Rohr, optische Eigenschaften wie die Transparenz oder Leitfähigkeit der Flüssigkeit. Die Regelung des Elektrolysestromes kann mit Hilfe von optischen Sensoren (z.B. Photozelle, Photodiode, Phototransistor, Photowiderstand) oder elektrischen Sensoren (Leitfähigkeits-, Kapazitätssensoren) oder anderen geeigneten Sensoren erfolgen.

In dem Verfahren mit Schaumbildung zur Anreicherung, Gewinnung oder Abtrennung von in einer Flüssigkeit gelösten oder emulgierten Pflanzenproteinen kann die Stromstärke oder Stromdichte während der elektrolytischen Wasserstoffgaserzeugung verändert werden. Bei kontinuierlicher Elektrolyse kann z.B. die Stromstärke vergrößert, verkleinert oder variiert werden. Es kann die Stromdichte während der Elektrolyse geregelt werden. Die Regelung kann wie für den Pulsbetrieb beschrieben erfolgen. Die Variation der Stromstärke kann mit einem Pulsbetrieb kombiniert werden. Beispielsweise kann die Stromdichte von Strompulsen variiert werden, z.B. eine Abfolge von Pulsen konstanter Stromstärke mit variabler Höhe von Puls zu Puls oder eine Abfolge von Pulsen variabler Stromstärke mit konstanter Höhe von Puls zu Puls oder eine Abfolge von Pulsen variabler Stromstärke mit variabler Höhe von Puls zu Puls.

Bei der Elektrolyse kann es zu Verschmutzungen und Belägen auf den Elektroden kommen. Daher kann es von Vorteil sein, während der Elektrolyse die Elektroden ein- oder mehrmals umzupolen. Für einen Elektrolysebetrieb mit Umpolung der Elektroden ist eine Ausrüstung der Elektrolysezelle mit geeigneten Elektroden oder ein symmetrischer Aufbau (gleiche Elektroden) der Elektrolysezelle vorteilhaft. Beispielsweise kann eine geteilte oder ungeteilte Elektrolysezelle für eine kathodische Wasserstoffentwicklung und eine anodische Sauerstoffentwicklung mit anodischer Oxidation von organischen Stoffen mit Platin-Elektroden für eine Umpolbarkeit bestückt werden.

Ein weiterer Gegenstand der Erfindung ist ein Verfahren zur Anreicherung, Gewinnung oder Entfernung von Pflanzenproteinen aus einer wässrigen Flüssigkeit, das dadurch gekennzeichnet ist, dass Wasserstoffgas elektrolytisch erzeugt wird und die Elektrolyse kontinuierlich oder diskontinuierlich, bei konstanter oder variabler Stromstärke, gesteuert, geregelt oder programmgesteuert, gepulst, mit Strompulsen oder mit Umpolung der Elektroden erfolgt.

Es werden bei einer Proteingewinnung, z.B. aus Kartoffelfruchtwasser, 80 bis 90 Prozent der Proteine aus der eingesetzten Flüssigkeit gewonnen. Überraschenderweise enthalten die aus Pflanzenfruchtwasser, insbesondere Kartoffelfruchtwasser, extrahierten Proteine bzw. das Produkt sehr geringe Mengen an Glykoalkaloiden. Hier ist das Verfahren den bekannten Verfahren überlegen. Die isolierten Proteine sind löslich. Das macht die mit Wasserstoffgas-Schaum gewonnenen Produkte für Anwendungen im Lebensmittelbereich sehr geeignet.

Die Abreicherung von Pflanzenproteinen einer Flüssigkeit mittels einer Schaumbildung, insbesondere mittels elektrochemisch erzeugtem Schaum, kann zur Reinigung von Flüssigkeiten aus Prozessen der Verarbeitung von Pflanzenprodukten genutzt werden.

In einem Verfahren zur Reinigung von Flüssigkeiten mit organischen Bestandteilen und Pflanzenproteinen wie Prozesswasser, Abwasser oder Pflanzenfruchtwasser wird vorteilhaft der Gehalt der Flüssigkeit an organischen Bestandteilen mittels Schaumbehandlung reduziert und die so abgereicherte Flüssigkeit einer weiteren Reinigung zugeführt. Die weitere Reinigung der abgereicherten Flüssigkeit erfolgt beispielsweise durch anodische Oxidation. Vorteilhaft wird die mittels Schaumbehandlung abgereicherte Flüssigkeit als Elektrolyt, insbesondere als Anolyt in einer Elektrolysezelle eingesetzt. Vorteilhaft können eine Gewinnung von Pflanzenproteinen mittels Schaumerzeugung durch kathodische Wasserstoffentwicklung aus einer Flüssigkeit mit Pflanzenproteinen (kathodische Schaumbehandlung) und eine Reinigung durch anodische Oxidation von organischen Bestandteilen einer Flüssigkeit, z.B. abgereicherte Flüssigkeit aus einer Zerschäumung oder Schaumbehandlung, in einer geteilten Elektrolysezelle oder weniger bevorzugt in einer ungeteilten Elektrolyse-Zelle parallel erfolgen. Ein weiterer Gegenstand der Erfindung ist ein Verfahren, in dem eine kathodische Schaumbehandlung einer Flüssigkeit und eine Reinigung einer anderen Flüssigkeit (z.B. abgereicherte Flüssigkeit) mittels einer direkten oder indirekten elektrochemischen Oxidation kombiniert werden. Eine direkte elektrochemische Oxidation ist eine anodische Oxidation. Eine indirekte elektrochemische Oxidation ist eine Oxidation durch ein Oxidationsmittel, das an der Anode gebildet wird, z.B. ein Redoxmediator. Der Einsatz eines Redoxmediators erfordert eine geteilte Elektrolyse-Zelle. Vorteilhaft ist die Anode eine Elektrode mit leitfähigem Diamant, z.B. eine BDD-beschichtete Elektrode.

Durch kathodische Schaumbehandlung (Zerschäumung) und nachfolgender oder paralleler anodischer Oxidation kann der CSB-Wert und BSB-Wert einer Flüssigkeit, die Pflanzenproteine enthält, stark verringert werden (CSB steht für Chemischer Sauerstoffbedarf, BSB für Biologischer Sauerstoffbedarf).

Das Reinigungsverfahren (kombiniertes Verfahren) wird in der Regel mit einer geteilten Zelle durchgeführt. Vorzugsweise trennt die Elektrodenräume eine Ionenaustauschermembran. Als Kathode wird eine für eine Wasserstoffentwicklung geeignete Elektrode eingesetzt. Als Anode wird eine Elektrode aus einem Elektrodenmaterial mit hoher Sauerstoffüberspannung bevorzugt, z.B. ein SnO₂ enthaltendes Elektrodenmaterial, spiegelglattes Platin oder leitfähiger Diamant wie BDD. Bevorzugt wird als Anode eine Elektrode, die aus einem leitfähigen Substrat (Nb, Ti ; korrosionsbeständiges Metall oder Metalllegierung; Metallsubstrate) in Form eines Streckmetalls besteht und mit Bor dotierten Diamant beschichtet ist. Das Verfahren kann beispielsweise mit einer Durchflußzelle betrieben werden. In diesem Fall wird die Flüssigkeit, z.B. frisches Kartoffelfruchtwasser, in den Kathodenraum (als Katholyt) und abgereicherte Flüssigkeit (verbrauchter Katholyt) in den Anodenraum eingespeist. Das kann jeweils mittels einer Pumpe aus einem Reservoir erfolgen. Eine Verwendung einer geteilten Zelle mit Ionenaustauschermembran und einer "zero-gap"-Anordnung der Elektroden (auf den beiden Seiten der Ionenaustauschermembran direkt angeordnete Elektroden, z.B. Gitter-Elektroden) ist besonders vorteilhaft.

Als Kathode dient z.B. eine Grafitplatte und als Anode eine Elektrode mit leitfähiger Diamant-Beschichtung (z.B. BDD, bordotierter Diamant). Vorteilhaft wird über dem Kathodenraum ein dicht anschließendes Rohr (z.B. 0,3 m, 0,5 m, 1 m oder 2 m lang) angeordnet, worin gebildeter Schaum aufsteigen kann. Beispielsweise beträgt die Pumpgeschwindigkeit bei einem Verfahren zur Reduzierung der organischen Bestandteile in einer Flüssigkeit (Verfahren zur Reinigung einer Flüssigkeit), bei dem die Flüssigkeit kathodisch und anodisch behandelt wird, bei Katholyt: 5 ml/min und bei Anolyt: 1 I/min. Im Kreislaufbehälter mit z.B. Kartoffelfruchtwasser oder kathodisch abgereichertem Kartoffelfruchtwasser als Anolyt stellt sich in der Regel bald ein pH-Wert kleiner als 3 ein. Es kommt hier zur Ausfällung von Organik und zur Klärung der Lösung. Bei einem Verfahren mit kathodischer und anodischer Behandlung einer Flüssigkeit wie Pflanzenfruchtwasser im Durchflussbetrieb werden die Durchflussmengen der Elektrolyte vorteilhaft so eingestellt, dass die Verweilzeit des Anolyten in dem Anodenraum kleiner als die Verweilzeit des Katholyten im Kathodenraum ist. Besonders vorteilhaft ist die Verweilzeit des Anolyten in dem Anodenraum fünf bis zehnmal kleiner, vorzugsweise zehnmal kleiner, als die Verweilzeit des Katholyten im Kathodenraum.

### Beispiele

### 1. Proteingewinnung mit Elektrolyse-Zelle im Batch-Betrieb

Die Elektrolyse-Zelle enthält einen Elektrodenstapel aus aufeinander gelegten Kathoden- und Anoden-Gitterelektroden, die mit Kunststoffgitter-Abstandshaltern (Abstand d=1mm) voneinander isoliert sind. Die Kathoden bestehen aus Edelstahlnetzen, die Anoden aus mit bordotiertem Diamant beschichteten Streckmetallelektroden aus Niob. Der Behälter der Elektrolyse-Zelle ist in diesem einfachsten Falle ein Becherglas mit einem Volumen von 2,5 Liter. Die wirksame Fläche einer einzelnen Elektrode beträgt 120cm². Im Elektrodenstapel sind abwechselnd zwei Anoden mit drei Kathoden verbaut. Die gesamte Kathodenfläche beträgt 360 cm².

Es wird ein Volumen von 1,8 Liter Kartoffelfruchtwasser eingefüllt und mäßig mittels eines Magnetstabs gerührt. Die Batch-Elektrolyse erfolgte bei einer Anfangstemperatur von 15° C und einer Endtemperatur von 22,5° C nach 10 Stunden Betrieb. Der Strom betrug konstant 3,6A und die mittlere Zellspannung 1,7 V. Der pH-Wert wurde auf pH 5 eingestellt und konstant gehalten. Zur Aufarbeitung wurde der Proteinschaum, der sich auf der Flüssigkeitsoberfläche bildete, abgeschöpft und im Rotationsverdampfer unter Teilvakuum getrocknet. Es wurden 4,5 g eines hellgelben bis weißlichgelben Pulvers erhalten, das nach Stickstoffbestimmung nach Kjehldahl weitgehend aus Protein besteht.

### 2. Elektrolyse mit Umpolung der Elektroden

Es wird eine einfache Elektrolysezelle verwendet, die aus einem Labor-Becherglas (graduiertes Volumen: 800 ml, Höhe: 14 cm, Durchmesser:11 cm) als Elektrolyse-Behälter und aus zwei Elektroden aus platiniertem Titanstreckmetall (Höhe: 6 cm, Breite: 10 cm) als Kathode und Anode besteht.

In diesem Beispiel ist der Weg, den die Blasen durch die Lösung nehmen müssen, allein durch die Elektrodenhöhe bestimmt. Die Höhe des gebildeten Schaumes über der Flüssigkeit beträgt 3,5 cm.

Als Elektrolyt wird ein Kartoffelfruchtwasser mit einem Gehalt von 1,2 Gewichtsprozent (% w/w) Protein (berechnet aus der Stickstoff-Bestimmung nach Kjehldahl) eingesetzt. Diese Lösung wird mittels einer Schlauchpumpe aus einem Reservoir (Volumen: 5 Liter) langsam in das Becherglas gepumpt und mit der gleichen Pumpe derart abgesaugt, dass sich das Flüssigkeitsniveau 1 cm über den Elektroden einstellt.

Die Elektroden werden an einer regelbaren Gleichstromquelle angeschlossen. Die Elektrolyse erfolgt bei einer konstanten Stromstärke von 0,9 A. Die Anfangsklemmenspannung (Zellspannung) beträgt 1,5 V. Mit diesen Einstellungen ist eine theoretische Gesamtversuchszeit von 83 Stunden notwendig.

Der pH-Wert wurde auf pH=5 eingestellt und konstant gehalten.

Nach einer Betriebszeit von 20h war die Klemmenspannung um 250% angestiegen. Der Grund liegt in der beginnenden Verblockung der Elektroden (Deckschichtbildung an den Elektroden). Als Gegenmaßnahme wurden nun die Elektroden in einem Zeitabstand von 0,5 Stunden für 2 Minuten umgepolt. Nach 3 Stunden war die Klemmenspannung auf 130% des Anfangswertes gesunken und blieb während der restlichen Betriebszeit konstant. Nach einer Betriebszeit von 50 Stunden wurde der Gleichrichter von den Elektroden getrennt und der gesammelte Schaum gewogen und in geeigneter Form eine Stickstoff-Bestimmung nach Kjehldahl durchgeführt. Der Elektrolyt wurde vor und am Ende der Elektrolyse mittels einer Stickstoff-Bestimmung nach Kjehldahl analysiert.

In diesem Experiment ergibt sich ein Anreicherungsfaktor von 6,5 (Verhältnis von Proteingehalt im Schaum und Proteingehalt der ursprünglichen Flüssigkeit), das heißt, daß das Protein im Schaum um das sechseinhalbfache aufkonzentriert worden ist. Aus den Untersuchungen der Lösungen ergab sich, daß 60% des Proteins der ursprünglich eingesetzten Flüssigkeit im Schaum wieder gefunden wurde.

### 3. Elektrolyse-Zelle mit Kanal für aufsteigenden Schaum

Die Elektrolyse-Zelle besteht im wesentlichen aus einem Schacht (Höhe: 100 cm; Grundfläche: 0,12 cm Breite, 5,5 cm Tiefe). In diesen Schacht werden Titan-Streckmetallelektroden, die in einem Rahmen auf einen Abstand von 2 cm fixiert wurden, eingeschoben. Die Streckmetall-Anode ist mit einem Gemisch aus Edelmetalloxiden beschichtet. Die Kathode ist unbeschichtet. Die Elektrodenfläche beträgt jeweils 275 cm². Nach Befüllung der Elektrolyse-Zelle mit der Flüssigkeit bleibt ein freier Kanal von etwa 0,5 m Länge, in dem der Schaum aufsteigen kann. Die Durchmischung des Elektrolyten wird dadurch erreicht, dass man mittels einer peristaltischen Pumpe Elektrolyt aus dem oberen Flüssigkeitsbereich der Elektrolyse-Zelle absaugt und in den unteren Bereich zurückführt.

Im Betrieb steigt der Schaum je nach Konsistenz säulenartig etwa 3 bis 10 cm über die Kante des Zellengehäuses (oberes Ende des Schachtes), bis die Schaumkrone bricht. Der herunterfallende Schaum wird in einer Sammelrinne aufgefangen, gesammelt und unter Vakuum bei 25-30°C getrocknet.

3,4 Liter Kartoffelfruchtwasser werden in die Elektrolyse-Zelle eingefüllt. Die Elektroden werden an einen Gleichrichter angeschlossen. Es fließt ein konstanter Elektrolysestrom von 2,2A. Daraus errechnet sich eine Stromdichte von i=8mA/cm². Nach einer Elektrolysezeit von 4,7 Stunden wurden, wie oben beschrieben, aus dem gesammelten Schaum durch Vakuumtrocknen 1,17g Pulver isoliert und ein Proteingehalt von 88% bestimmt (mittels Stickstoff-Bestimmung nach Kjehldahl).

### 4. Zerschäumung mit Wasserstoffgas in geteilter Zelle

Es wird eine geschlossene Elektrolyse-Zelle eingesetzt, die durch eine Kationenaustauschermembran in ein kathodisches und ein anodisches Kompartiment geteilt ist und mit Platinelektroden ausgerüstet wurde. Das anodische Kompartiment wird mit verdünnter Essigsäure befüllt. Das kathodische Kompartiment ist mit dem Bodenauslaß eines Vorratsgefäßes verbunden, in das das Kartoffelfruchtwasser mit pH 5 eingefüllt wurde. Ein Rohr führt aus dem oberen Teil der Elektrolyse-Zelle heraus und ist so angeordnet, dass der darin transportierte Schaum von oben in ein Sammelgefäß fällt. In einem Pumpkreislauf mit peristaltischer Pumpe wird aus dem Vorratsgefäß Lösung abgepumpt und in den unteren Teil der Zelle eingespeist. Die Gasblasen werden, gemessen an der Elektrodenhöhe, durch einen relativ verlängerten Weg durch die Flüssigkeit (Flüssigkeitssäule über den Elektroden) geführt und auch die sich ausbildende Schaumsäule ist im Vergleich zu den vorigen Ausführungen verlängert. Es besteht ein Längenverhältnis Elektrodenlänge:Flüssigkeitssäule:Schaumsäule von etwa 1:3,3:5.

An die identischen Platinelektroden wird eine Stromdichte von 7,7 mA/cm² angelegt.

Der Schaum, der am oberen Ende des Rohres austritt, wird aufgefangen und analysiert wie vorbeschrieben. Die aus dem Schaum gewonnene Trockensubstanz hat einen Proteingehalt von 92% (mittels Stickstoff-Bestimmung nach Kjehldahl).

### Vergleichsbeispiel

Ein Standzylinder (Höhe: 1m, Durchmesser: 8 cm) wird mit 2,5 Liter Kartoffelfruchtwasser befüllt. Durch eine Fritte mit D3-Porosität wird Luft auf dem Boden des Gefäßes eingeperlt. Es ergibt sich eine Flüssigkeitssäule mit einer Höhe von 50 cm und eine Schaumhöhe von 50 cm. Das Gas wird mit einer Gasflußgeschwindigkeit von v=30ml/min eingespeist. Im Gegensatz zu den Resultaten der vorigen Versuche enthielt der Schaum sehr große Blasen und wies insgesamt eine sehr geringe Dichte auf. Er ist unter technischen Bedingungen nur schwierig in einem Produktionsprozess weiter zu bearbeiten. Der Gehalt an Trockensubstanz ist sehr gering. Es wurde ein Protein-Anreicherungsfaktor von 1,3 im Schaum ermittelt.

## Patentansprüche

1. Verfahren zur Anreicherung, Gewinnung oder Entfernung von Pflanzenproteinen aus einer wässrigen Flüssigkeit, **dadurch gekennzeichnet, dass** Gasblasen aus Wasserstoffgas oder einem Wasserstoffgas enthaltenden Gasgemisch oder elektrochemisch Gasblasen in der Flüssigkeit erzeugt werden und dadurch ein Schaum gebildet wird, der gelöste Pflanzenproteine enthält, und Schaum von der Flüssigkeit getrennt wird.

2. Verfahren nach Anspruch 1, **dadurch gekennzeichnet, dass** die Gasblasen in der Flüssigkeit in einer ungeteilten oder geteilten Elektrolyse-Zelle elektrochemisch erzeugt werden.

3. Verfahren nach einem der vorhergehenden Ansprüche, **dadurch gekennzeichnet, dass** die Gasblasen an mindestens einer Elektrode, enthaltend Platin, Iridium, Ruthenium, Iridium und Ruthenium, Grafit, leitfähiges Kohlenstoffmaterial oder leitfähigen Diamant, erzeugt werden.

4. Verfahren nach einem der vorhergehenden Ansprüche, **dadurch gekennzeichnet, dass** ein flüssiges Produkt oder Zwischenprodukt aus dem von der Flüssigkeit getrennten Schaum gewonnen wird, das einen höheren Gehalt an Pflanzenproteinen enthält als die ursprünglich eingesetzte Flüssigkeit.

5. Verfahren nach einem der vorhergehenden Ansprüche, **dadurch gekennzeichnet, dass** eine Vorrichtung eingesetzt wird, die mindestens ein Rohr, ein rohrartiges Gebilde, einen Hohlkörper mit einer Eingangsöffnung und einer Ausgangsöffnung oder einen Bereich aufweist, worin der gebildete Schaum aufsteigen kann, wobei die Vorrichtung eine Elektrolyse-Zelle oder eine Vorrichtung mit einer Einrichtung zur Gasblasenerzeugung ist.

6. Verfahren nach einem der vorhergehenden Ansprüche, **dadurch gekennzeichnet, dass** aus der Flüssigkeit eine von Pflanzenproteinen abgereicherte Flüssigkeit erzeugt wird, die einer direkten oder indirekten elektrochemischen Oxidation unterzogen wird.

7. Verfahren nach einem der vorhergehenden Ansprüche, **dadurch gekennzeichnet, dass** in einer geteilten Elektrolyse-Zelle kathodisch die Gasblasen in der Flüssigkeit zur Entfernung von Pflanzenproteinen erzeugt werden und anodisch eine von Pflanzenproteinen abgereicherte Flüssigkeit oder eine Flüssigkeit, die organische Stoffe enthält, einer direkten oder indirekten elektrochemischen Oxidation unterzogen wird.

8. Verfahren nach einem der vorhergehenden Ansprüche, **dadurch gekennzeichnet, dass** die eingesetzte Flüssigkeit ein Prozesswasser oder Abwasser aus der Verarbeitung von Pflanzen, Pflanzenteilen oder pflanzlichen Produkten oder die eingesetzte Flüssigkeit Pflanzenfruchtwasser oder Kartoffelfruchtwasser ist.

9. Verfahren nach einem der vorhergehenden Ansprüche, **dadurch gekennzeichnet, dass** die Gasblasen in der Flüssigkeit in einer ungeteilten oder geteilten Elektrolyse-Zelle elektrochemisch erzeugt werden, wobei kathodisch Wasserstoffgas und anodisch Sauerstoffgas entwickelt werden oder kathodisch Wasserstoffgas erzeugt und anodisch Wasserstoffgas oxidiert wird.

10. Verfahren nach einem der vorhergehenden Ansprüche, **dadurch gekennzeichnet, dass** die Flüssigkeit einen pH-Wert im Bereich von pH 4 bis pH 7 zu Beginn der Erzeugung der Gasblasen aufweist und der pH-Wert der Flüssigkeit konstant gehalten oder verändert wird.

11. Verfahren nach einem der vorhergehenden Ansprüche, **dadurch gekennzeichnet, dass** die Gasblasen elektrochemisch durch Elektrolyse erzeugt werden und die Elektrolyse kontinuierlich oder diskontinuierlich, gepulst oder mit mindestens einer Umpolung der Elektroden betrieben wird.

12. Verwendung von Wasserstoffgas oder einem Wasserstoffgas enthaltenden Gasgemisch zur Erzeugung von Schaum in einer Flüssigkeit, die Pflanzenproteine enthält, in einem Verfahren zur Gewinnung von Pflanzenproteinen aus der Flüssigkeit, zur Reinigung der Flüssigkeit oder zur Abreicherung von Pflanzenproteinen in der Flüssigkeit mittels einer Schaumbehandlung.

13. Verwendung nach Anspruch 12, **dadurch gekennzeichnet, dass** das Wasserstoffgas oder das Wasserstoffgas enthaltende Gasgemisch in der Flüssigkeit elektrochemisch erzeugt wird.

14. Verwendung nach Anspruch 12 oder 13, **dadurch gekennzeichnet, dass** die Flüssigkeit ein Pflanzenfruchtwasser oder Kartoffelfruchtwasser ist.

## Claims

1. A process for the enrichment, recovery or removal of plant proteins from an aqueous liquid, wherein gas bubbles of hydrogen gas or a hydrogen gas containing gas mixture or electrochemically generated gas bubbles are generated in the liquid, forming a foam that contains dissolved plant proteins, and foam is separated from the liquid.

2. A process according to claim 1, wherein the gas bubbles are generated electrochemically in the liquid in an undivided or divided electrolytic cell .

3. A process according to any one of the preceding claims, wherein the gas bubbles are produced on at least one electrode containing platinum, iridium, ruthenium, iridium and ruthenium, graphite, conductive carbon material or conductive diamond.

4. A process according to any one of the preceding claims, wherein a liquid product or intermediate product is recovered from the foam separated from the liquid, which contains a higher content of plant proteins than in the initial liquid.

5. A process according to any one of the preceding claims, wherein a device is used, which comprises at least a tube, a tubular structure, a hollow body having an inlet opening and an outlet opening or a region in which the formed foam can rise, where the device is an electrolytic cell or a device with an entity for gas bubble generation.

6. A process according to any one of the preceding claims, wherein a liquid depleted of plant proteins is produced from the liquid, which is thereafter subjected to a direct or indirect electrochemical oxidation.

7. A process according to any one of the preceding claims, where in a divided electrolytic cell gas bubbles are generated cathodically in the liquid to remove plant proteins and anodically a liquid depleted from plant proteins or a liquid containing organic substances is subjected to a direct or indirect electrochemical oxidation.

8. A process according to any one of the preceding claims, wherein the liquid used is a process water or wastewater from the processing of plants, parts of plants or plant products or the liquid used is plant fruit water or potato fruit water.

9. A process according to any one of the preceding claims, where the gas bubbles are produced electrochemically in the liquid in an undivided or divided electrolytic cell, where hydrogen gas is generated at the cathode and oxygen gas at the anode or hydrogen gas is generated cathodically and hydrogen gas is oxidized anodically.

10. A process according to any one of the preceding claims, wherein the liquid has a pH value in the range of pH 4 to pH 7 at the beginning of the generation of gas bubbles and the pH of the liquid is kept constant or changes.

11. A process according to any one of the preceding claims, wherein the gas bubbles are produced electrochemically by electrolysis and the electrolysis is operated continuously or discontinuosly, pulsed or with at least one reversal of the polarity of the electrodes.

12. The use of hydrogen gas or a gas mixture containing hydrogen gas for producing foam in a liquid containing plant proteins in a process for recovery of plant proteins from the liquid, for purification of the liquid or for depletion of plant proteins from the liquid by foam treatment.

13. The use according to claim 12, wherein the hydrogen gas or the hydrogen gas containing gas mixture is produced electrochemically in the liquid.

14. The use according to claim 12 or 13, wherein the liquid is a plant fruit water or potato fruit water.

## Revendications

1. Procédé pour l'enrichissement, l'obtention ou élimination de les protéines végétales dans un liquide aqueux, **caractérisé en ce que** des bulles de gaz de l'hydrogène gazeux ou un mélange gazeux contenant de l'hydrogène gazeux ou des bulles de gaz générées par voie électrochimique sont générées dans le liquide et ainsi se forme une mousse contenant des protéines végétales dissoutes, et la mousse est séparée du liquide.

2. Procédé selon la revendication 1, **caractérisé en ce que** les bulles de gaz sont générées dans le liquide par voie électrochimique dans une cellule d'électrolyse non divisée ou divisée.

3. Procédé selon l'une quelconque des revendications précédentes, **caractérisé en ce que** les bulles de gaz sont produites sur au moins une électrode contenant le platine, l'iridium, le ruthénium, l'iridium et le ruthénium, le graphite, le matériau carboné conductrice ou diamant conductrice.

4. Procédé selon l'une quelconque des revendications précédentes, **caractérisé en ce qu'**un produit liquide ou un produit intermédiaire est récupéré à partir du liquide séparé de la mousse contenant une plus forte teneur en protéines végétales que le liquide utilisé initialement.

5. Procédé selon l'une quelconque des revendications précédentes, **caractérisé en ce qu'**un dispositif est utilisé qui présente au moins un tuyau, une structure en forme de tube, un corps creux ayant une ouverture d'entrée et une ouverture de sortie ou une zone dans laquelle la mousse formée peut s'élever, dans lequel le dispositif est une cellule électrolytique ou un dispositif avec une entité pour la génération de bulles de gaz.

6. Procédé selon l'une quelconque des revendications précédentes, **caractérisé en ce qu'**un liquide appauvri en protéines végétales est produite à partir du liquide, qui est ensuite soumis à une oxydation électrochimique directe ou indirecte.

7. Procédé selon l'une quelconque des revendications précédentes, **caractérisé en ce que** dans une cellule d'électrolyse divisée les bulles de gaz sont générées cathodiquement dans le liquide pour éliminer les protéines végétales et à l'anode un liquide appauvri en protéines végétales ou un liquide contenant des substances organiques est soumis à une oxydation électrochimique directe ou indirecte.

8. Procédé selon l'une quelconque des revendications précédentes, **caractérisé en ce que** le liquide utilisé est une eau de traitement ou des eaux usées de la transformation de plantes, de parties de plantes ou de produits végétaux ou les liquides utilisés sont liquides contenant jus de plantes ou jus de pomme de terre.

9. Procédé selon l'une des revendications précédentes, **caractérisé en ce que** les bulles de gaz sont produites dans le liquide par voie électrochimique dans une cellule d'électrolyse non divisée ou divisée, où le gaz d'hydrogène est généré à la cathode et de l'oxygène gazeux à l'anode ou le gaz d'hydrogène est généré à la cathode et d'hydrogène est oxydé à la anode.

10. Procédé selon l'une quelconque des revendications précédentes, **caractérisé en ce que** le liquide a une valeur de pH dans la plage de pH 4 à pH 7 au début de la génération de bulles de gaz et le pH du liquide est maintenu constant ou modifié.

11. Procédé selon l'une quelconque des revendications précédentes, **caractérisé en ce que** les bulles de gaz sont générées par voie électrochimique par électrolyse et l'électrolyse est continu ou discontinu, pulsé ou exploités avec au moins une inversion de polarité des électrodes.

12. Utilisation de l'hydrogène gazeux ou un mélange gazeux contenant de l'hydrogène gazeux de produire une mousse dans un liquide contient des protéines végétales dans un procédé pour obtenir des protéines végétales à partir du liquide, pour le purification de la liquide ou pour l'appauvrissement des protéines végétales dans le liquide au moyen d'un traitement de mousse.

13. Utilisation selon la revendication 12, **caractérisé en ce que** l'hydrogène gazeux ou un mélange gazeux contenant de l'hydrogène gazeux est produit par voie électrochimique dans le liquide.

14. Utilisation selon la revendication 12 ou 13, **caractérisé en ce que** le liquide est un liquide contenant jus de plantes ou jus de pomme de terre.
